# EUROPEAN PATENT APPLICATION

(11) **EP 3 982 373 A1**
(43) Date of publication of application: **13.04.2022**
(21) Application number: 20200616.9
(22) Date of filing: 07.10.2020
(51) Int. Cl.: G16H 20/70, G09B 5/00, G09B 7/02, G09B 7/00

(54) **APPARATUS TO ASSIST A USER TO REMEMBER THE IDENTITY OF A PERSON**

(71) Applicant: Koninklijke Philips N.V., 5656 AG Eindhoven (NL)
(72) Inventor: SPEKOWIUS, Gerhard Reinhold, 5656 AE Eindhoven (NL); KLAMING, Laura, 5656 AE Eindhoven (NL); KARAKAYA, Koray, 5656 AE Eindhoven (NL)
(74) Representative: Philips Intellectual Property & Standards

(57) **Abstract**

According to an aspect, there is provided an apparatus (200) comprising a processor (102). The processor (102) is configured to receive an image of at least the face of a person whose identity is to be remembered; receive a name of the person whose identity is to be remembered; extract an image feature from the facial image; select, from a group of images, an image associated with the image feature; extract a name feature from the name; associate the name feature with the selected image; and provide the selected image and the name feature for presentation to a user. A computer-implemented method and a computer program product are also disclosed.

## Description

### FIELD OF THE INVENTION

The invention relates to remembering the identity of a person and, more particularly, an apparatus and a method for assisting a user to remember an association between the name and the face of a person.

### BACKGROUND OF THE INVENTION

Compensatory cognitive training can be used to help people with subjective cognitive decline (i.e. increased forgetfulness). Research shows that compensatory cognitive training can be used to improve memory performance in healthy people, people with neurological or psychiatric diseases, and people with other causes of cognitive impairment. Compensatory cognitive training builds on cognitive strategies, with many cognitive strategies relying on visual processes and semantic memory.

Several techniques exist for improving a person's memory. For example, a user may associate information that they wish to remember with something more accessible or memorable, such as an image. A person may be able to remember physical objects more easily than abstract or textual information by being able to relate more easily to the physical objects, which may invoke stronger emotions than purely textual information. Thus, the use of images may help a person to retain and recall associations between a physical object and an abstract or textual piece of information.

It can be difficult for people to generate associations between abstract or textual information and a physical object, especially for those with subjective cognitive decline. There is therefore a need for an apparatus which is able to assist an individual in creating such associations.

### SUMMARY OF THE INVENTION

Compensatory cognitive training has been shown to help people with subjective cognitive decline; for example, by improving their memory performance. Some cognitive strategies rely on visual processes and semantic memory to improve the memory of those experiencing cognitive impairment. A mnemonic is one way in which a person's memory may be improved, which can involve making associations between information that a person can remember more easily, such as physical objects, and information that is less easy to remember, such as textual information. The inventors of the present disclosure have recognized that the process of generating associations to aid a person's memory can be automated and optimized to make it easier for a person to associate someone's name with their face. According to embodiments disclosed herein, a person's ability to associate someone's name with their face is achieved by extracting a distinctive feature from an image of their face, extracting a feature from the name, then based on the extracted facial feature, retrieving an image of something that the user is more likely to be able to remember, and associating that image with the feature extracted from the name.

According to a first specific aspect, there is provided an apparatus comprising a processor. The processor is configured to receive an image of at least the face of a person whose identity is to be remembered; receive a name of the person whose identity is to be remembered; extract at least one image feature from the image of the face; select, from a set of stored images, a first image associated with the at least one image feature; extract first name feature from the name; associate the first name feature with the selected first image; and provide the selected first image and the first name feature for presentation to a user.

In some embodiments, the processor may be further configured to select, from the set of stored images, a second image associated with the at least one image feature; associate the first name feature with each of the first image and the second image; and provide the selected first image, the selected second image and the first name feature for presentation to the user.

The processor may, in some embodiments, be further configured to extract a second name feature from the name; associate the first name feature and the second name feature with the selected first image; and provide the selected first image, the first name feature and the second name feature for presentation to the user.

In some embodiments, the images in the set of images may be grouped according to a plurality of categories. The processor may be configured to select the first image associated with the at least one image feature by selecting the first image from a user-selected category of the plurality of categories.

The processor may, in some embodiments, be further configured to compare the association between the at least one name feature and the selected image with at least one previously-made association; and responsive to determining that the association matches a previously-made association, select, from the set of stored images, a different image associated with the name feature.

In some embodiments, the processor may be configured to select the first image associated with the at least one image feature by searching the set of stored images to identify an image with a feature that matches, or is similar to, the at least one image feature.

In some embodiments, each image in the set of stored images may have at least one image feature of a defined list of distinctive image features.

In some embodiments, the at least one image feature may comprise a facial landmark feature.

In some embodiments, the at least one name feature may comprise one or more of the whole name, part of the name, a homophone of the name or part of the name, a word that rhymes with the name or part of the name, and an anagram of the name or part of the name.

The apparatus may further comprise, in some embodiments, a storage device for storing at least one of the received image, the received name, the set of stored images, and the association of the at least one name feature with the selected image; and a user interface for presenting the selected first image and the name feature to the user.

According to a second aspect, there is provided a computer-implemented method comprising receiving an image of at least the face of a person whose identity is to be remembered; receiving a name of the person whose identity is to be remembered; extracting at least one image feature from the image of the face; selecting, from a set of stored images, a first image associated with the at least one image feature; extracting at least one name feature from the name; associating the at least one name feature from the name with the selected first image; and providing the selected first image and the name feature for presentation to a user.

In some embodiments, a predictive model may be used to perform at least one of the steps of: extracting the at least one image feature, selecting the first image, and extracting at least one name feature.

The computer-implemented method may further comprise, in some embodiments, receiving a user input indicating a category of a plurality of categories. In some embodiments, each image in the set of stored images may be associated with at least one of the plurality of categories. In some embodiments, selecting a first image associated with the at least one image feature may comprise selecting a first image from the category indicated in the user input.

In some embodiments, extracting at least one image feature from the image may comprise extracting a plurality of image features from the image of the face; presenting the plurality of image features to the user; receiving a user-selection of one of the plurality of image features; and using the user-selected image feature as the extracted image feature.

According to a third aspect, there is provided a computer-program product comprising a non-transitory computer-readable medium, the computer-readable medium having computer-readable code embodied therein, the computer-readable code being configured such that, on execution by a suitable computer or processor, the computer or processor is caused to perform steps of the method disclosed herein.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic illustration of an example of a system for associating the name and face of a person;
Fig. 2 is a schematic illustration of an example of an apparatus for associating the name and face of a person;
Fig. 3 is a flowchart of an example of a method for associating the name and face of a person;
Fig. 4 is a flowchart of a further example of a method for associating the name and face of a person; and
Fig. 5 is a schematic illustration of a processor in communication with a non-transitory computer readable medium.

### DETAILED DESCRIPTION OF EMBODIMENTS

Embodiments of the present invention provide a mechanism to assist a user in remembering a person's name or, more specifically, to assist a user in associating the name of a person with that person's face. The disclosed embodiments make use of imagery to supplement an image of the person whose identity is to be remembered, and can provide support during compensatory cognitive training to help people with cognitive impairment, such as subjective cognitive decline. More generally, embodiments disclosed herein can help a person to remember things more reliably.

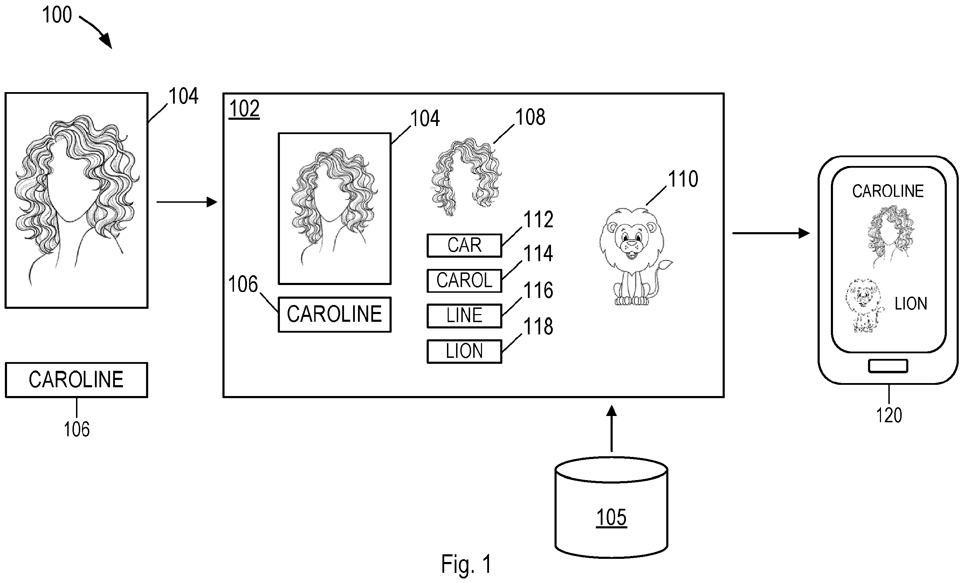

Fig. 1 is a schematic illustration of an example of a system 100 for associating the name and face of a person. The system 100 includes a processor 102 or processing apparatus, which may, for example, form part of an apparatus, such as computing device (e.g. a desktop computer, a laptop computer, a tablet computer, a smart phone, a wearable device, or a server). As will become apparent from the discussion below, the processor 102 is configured to perform the various processing steps involved with the methods disclosed herein. The processor 102 receives, as an input, an image 104, such as an image of a person. The image 104, which may be provided in any suitable form (e.g. JPEG, bitmap, or the like) may be acquired using a camera. The camera may be a standalone imaging device, a video camera, a camera located in a smart phone, a closed-circuit television camera, or the like. In some examples, the processor 102 may be configured to obtain the image 104. For example, the image 104 may be acquired from a storage medium, such as a storage medium located on a mobile phone, a smart phone, a computing device, or a remote server, or from the cloud, the internet, or the like. The storage medium may comprise a hard drive, random access memory, or the like. The image 104 may comprise an image of a person's face, a person's head, a person's body, or the like, but, typically, the image includes at least the face of a person whose identity is to be remembered. The processor 102 also receives, as an input, the name 106 of the person whose identity is to be remembered. In some examples, the name 106 of the person may be provided to the processor 102 along with the image 104 of the person (e.g. as part of the same file, or as separate but associated/related files) while, in other examples, the processor 102 may be configured to obtain the name 106 (e.g. by performing an interrogation of data or by generating a request for the name). The name 106 may be supplied to the processor 102 by a user, for example via a user interface. The user may supply the name to the device via a keyboard, a touch-screen display, a microphone, or the like. Sound input via a microphone may be converted into text using by the processor 102. In an example use case, a user may meet a person for the first time, and may use the system 100 to aid their memory of the identity of the person. The user may, for example, capture a photograph (e.g. the image 104) of the person using their smartphone (e.g. via a dedicated memory assistance application), and enter the person's name 106 using the smartphone keyboard. In this example, the person's name 106 is "Caroline".

The processor 102 may be configured to extract at least one image feature 108 from the image 104. For example, the processor 102 may be configured to analyze the image 104 and identify one or more features of the image that stand out, or may be considered to be distinctive. In some embodiments, facial recognition techniques may be used to detect image features 108 appearing in the image 104 that correspond to known facial features, or facial landmark features. Thus, the at least one image feature 108 extracted from the image 104 may comprise a facial landmark feature. For example, the processor 102 may be configured to identify a distinctive feature appearing in the image 102 from a defined list of distinctive features, such as a color of the person's eyes (e.g. blue eyes), the size of a facial feature (e.g. a large nose or large ears), a distinctive feature relating to the person's hair (e.g. frizzy hair, long curly hair, spiky hair, or a bald head), and so on. In other examples, the processor 102 may be configured to extract any other image feature 108 from the image 104 of the person including, for example, a portion of the image 104 showing a scar, a piece of jewelry (e.g. an ear ring or a nose piecing), a pair of glasses, or the like. Thus, the image feature 108 may comprise a portion of the image 104 corresponding to a feature of the person and, particularly, a feature of the person that may be considered to be distinctive (e.g. according to a defined list of distinctive features). In the example shown in Fig. 1, the image feature 108 extracted from the image 104 is the portion of the image corresponding to the person's hair (i.e. long curly hair).

The image feature 108 extracted from the image 104 is used as the basis of a search for a different image that the user may find more memorable, and which the user may associate with the person whose identity they are trying to remember to assist their memory of the identity.

The processor 102 is configured to select, from a set of stored images, a first image associated with the at least one image feature. For example, a set of images may be stored in a database 105 accessible by the processor 102. The database 105 may be located in a storage device within the same apparatus as the processor 102, or in a storage device remote from, but in contact with, the processor. Alternatively, the processor 102 may be configured to select a first image from another source of images, such as an online database (e.g. the Internet). In some examples, the processor 102 may be configured to select an image (i.e. a first image associated with the at least one image feature) by searching the set of stored images to identify an image with a feature that matches, or is similar to, the at least one image feature. Each image in a set of stored images may have at least one image feature of a defined list of distinctive image features. In some examples, an image in the set of stored images may have multiple distinctive image features (e.g. a stored image may show a person having short spiky hair and big ears and, therefore, that stored image may be identified and/or retrieved as a result of a search for the image feature "short spiky hair" or it may be retrieved as a result of a search of the image feature "big ears"). In the example shown in Fig. 1" the extracted image feature 108 is "long curly hair", so the processor 102 searches the database 105 and selects an image from the set of stored images that is related to long curly hair, which, in this case, is an animal with curly or voluminous hair, namely a lion 110, due to the male lion's mane. In another example, the processor 102 may retrieve an image of a well-known person (e.g. a celebrity) with long curly hair.

Images in the set of stored images may be categorized or tagged such that the processor 102 is able to restrict its search of the stored images to images of a particular category or type. The user of the system 100 may find it easier to remember or associate with a particular class or category of objects (e.g. animals), and the processor 102 may therefore favor the selection of images from that class or category (e.g. images of animals).

In addition to selecting a more memorable image (e.g. the image of a lion 110) associated with an image feature extracted from the image 104 of the person whose identity is to be remembered, the invention involves finding a more memorable way of remembering the person's name 106. The processor 102 is configured to extract a first name feature from the name 106. In the context of this disclosure, a name feature is a feature of the person's name which may be used to aid the user in remembering the name, or remembering an association between the name and the selected image, so that the user can more easily recall the name and identity of the person. The first name feature of the name 106 may, for example, comprise at least one of: the whole name, part of the name, a homophone of the name or part of the name, a word that rhymes with the name or part of the name, an anagram of the name or part of the name, a word that rhymes with the name or part of the name, or the like. For example, if the name "Caroline" is supplied to the processor 102, an extracted name feature based on part of the name 106 might be "car" 112, "carol" 114 and/or "line" 116, and an extracted name feature based on an anagram of part of the name might be "lion" 118. In some examples, the processor 102 may select a name feature (e.g. one the four name features illustrated in Fig. 1) at random, while in other examples, a plurality of name features may be extracted, and a selection of a name feature may be based on a user selection, a user preference, a category of name features (e.g. nouns, animals, pronouns), or the like. In some examples, a plurality of extracted name features may be presented to the user, and the user may select the name feature that they think will help them to remember the identity of the person most effectively. In the example shown in Fig. 1, name feature extracted based on anagram part of the name "Caroline" is used (i.e. the name feature "lion" 118), and this becomes the first name feature.

The processor 102 is configured to associate the first name feature 118 with the selected first image 110. in the example shown in Fig. 1, the association between the first name feature 118 (i.e. "lion") and the selected first image 110 is a relatively strong one, where the association may comprise an image of a lion and the word "lion". In an alternative example (not illustrated), the extracted first name feature of the name may be the name feature 112 (i.e. "car") and the first image associated with the at least one image feature may be an image of a lion. In this case, the association may comprise an image of a lion and the word "car". In another example, the extracted first name feature of the name may be the name feature 114 (i.e. "carol") and the extracted at least one image feature from the image may be large ears, leading to selection of a first image associated with the at least one image feature comprising an elephant, a celebrity with large ears, or the like. In this example, assuming the selected first image is an image of an elephant, an association would be made between the word "carol" and the image of the elephant. In some examples, several extracted name features may be presented to the user along with several candidate images selected from the set of stored images, so that the user is able to select a name feature, an image, and/or a combination of a name feature and an image that they feel would best help them remember the identity of the person.

In some embodiments, the step of associating the first name feature with the selected first image may comprise assigning a score to the association. For example, these scores may be generated by the processor 102. The processor 102 may score the associations based on a number of factors including, for example, the likeness of the first name feature and the selected first image (e.g. the word "lion" and an image of a lion in the example given above may score relatively highly). In other embodiments, a user of the system 100 may score the associations, which may depend on the strength of the association, how likely they are to remember the association, whether the association invokes an emotional response (e.g. amusement or sadness), or the like.

The strength of an association may depend on how well a user is able to connect to and/or memorize said association. As noted above, in some embodiments, the images in the set of stored images may be grouped according to a plurality of categories. Some users may find that they are able to connect more easily to certain groups or categories of images including, for example, celebrities (e.g. television personalities, movie stars, or the like), animals, monuments, buildings, countries, food items, or the like. In some examples, the processor 102 may be configured to select a first image associated with at least one image feature from an image of a face by selecting the first image from a user-selected category of the plurality of categories.

The processor 102 may be configured to provide the selected first image and the first name feature for presentation to a user. In some examples, the selected first image and the first name feature may be presented to a user via a device 120. The device 120 may comprise a mobile telephone (e.g. a smart phone), a desktop computer, a laptop computer, a tablet, a wearable device or the like. In some examples, the selected first image 110 and the first name feature 118 (or 112, 114, 116) may be displayed on a screen (e.g. on a display of the device 120). In some examples, the selected first image 110 and the first name feature 118 may be displayed side by side. In other examples, the selected first image 110 and the first name feature 118 may be superimposed (e.g. the first name feature may be positioned within the selected first image, the first name feature may overlay part or all of the selected first image, or the like). In some examples, the selected first image 110 may be displayed on a screen and the first name feature 118 may be communicated to the user audibly (e.g. via a speaker associated with device 120). In some examples, such as the example shown in Fig. 1, the selected first image 110 and the first name feature 118 may be presented to the user alongside the image 104 and the name 106 of the person whose identity is to be remembered.

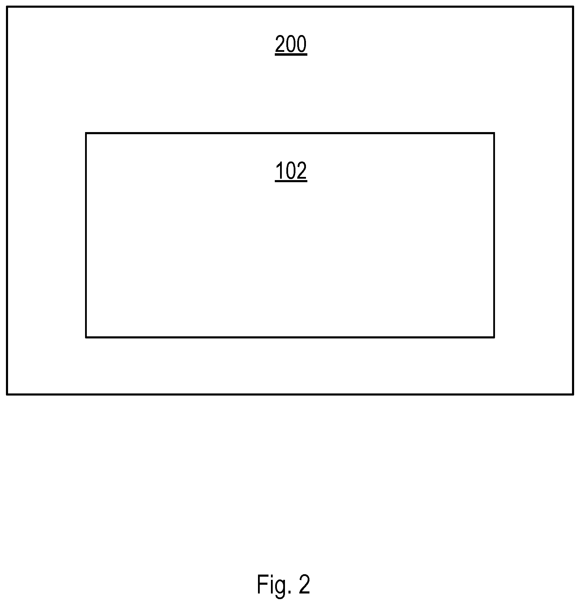

As noted above, the processor 102 of the system 100 discussed above may form part of an apparatus or device. According to a first aspect, an apparatus is provided. Fig. 2 is a schematic illustration of an example of an apparatus 200 comprising the processor 102. Thus, the processor 102 is configured to receive an image of at least the face of a person whose identity is to be remembered; receive a name of the person whose identity is to be remembered; extract at least one image feature from the image of the face; select, from a set of stored images, a first image associated with the at least one image feature; extract first name feature from the name; associate the first name feature with the selected first image; and provide the selected first image and the first name feature for presentation to a user.

As noted briefly above, a user may find it easier to remember some images than others and, similarly, may find it easier to remember some words or name features than others. Thus, in some embodiments, the processor 102 may be configured to select, from the set of stored images, a second image associated with at the least one image feature 108 extracted from the image 104 of at least the face of the person whose identity is to be remembered. The processor 102 may be configured to associate the first name feature with each of the first image 110 and the second image. In the example shown in Fig. 1, the first name feature (e.g. "lion" 118) may be associated with the selected first image 110 of a lion, and also with a selected second image (e.g. of waves reminiscent of the waves in the person's curly hair). The system 100 may be configured to provide the selected first image 110, the selected second image and the first name feature for presentation to the user. In this way, the user can select from multiple associations, and pick the association that they feel will help them to remember the identity of the person most effectively.

In other embodiments, the processor 102 may be configured to select, from the set of stored images, a plurality of images (e.g. three, four, five, ten, ten or more) associated with at least one image feature extracted from the image 104 of the face of a person whose identity is to be remembered (i.e. from the extracted image feature 108 or from one or more different image features extracted from the image 104). Advantageously, enabling the association of the first name feature with each of a first image and a second image (or additional images) allows a user to select an association from among a plurality of associations which will be most effective at helping them to remember the identity.

In some embodiments, multiple name features may be extracted from the name so that the user has multiple name features to associate with the selected image, or so that the user can select which name feature to associate with the selected first image, or so that multiple associations can be made, from which the user can select. For example, the processor 102 may be configured to extract a first name feature and a second name feature from the name. The processor 102 may be configured to associate the first name feature and the second name feature with the selected first image (i.e. the first image selected from a set of stored images). The processor 102 may be configured to provide the selected first image, the first name feature and the second name feature for presentation to a user. Advantageously, presenting multiple name features (e.g. "lion" and "car") to the user may provide for a stronger association between the selected first image and the name of the person whose identity is to be remembered.

In some embodiments, the processor 102 may be configured, in addition to extracting a first name feature from the name, and associating the first name feature with the selected first image, to extract a second name feature from the name, and to associate the second name feature with the selected second image. The processor 102 may provide the selected first image, the selected second image, the first name feature and the second name feature for presentation to the user. In some examples, the first name feature may be associated with the first selected image, and the second name feature may be associated with the second selected image while, in other examples, the first name feature may be associated with the second selected image, and the second name feature may be associated with the first selected image. As noted above, providing the user with multiple images that may aid their memory and multiple name features that may aid their memory may enable more memorable associations to be made.

Data provided to, used by and/or outputted by the processor 102 may be stored in a storage device associated with the processor. For example, in examples where the processor 102 is the processor of a smartphone, then data may be stored in a storage device of the smartphone. Such a storage device may be configured to store input images (e.g. the image 104), names (e.g. the name 106), previously-extracted image features (e.g. the image feature 108), previously-extracted name features (e.g. the name features 112, 114, 116, 118), previously-selected images from the set of stored images (e.g. the selected first image 110) and previously-made associations between extracted name features and selected images. In one example, each image 104 that is provided to the processor 102 may be stored in such a storage device. When a new image 104 of a person is provided to the processor 102, the storage device may be searched for candidate images that match or are similar to the new image 104, to see whether an association (i.e. of a name feature and a selected image) has previously been made in respect of the person. If a matching/similar images are identified, then the previous association may be retrieved and presented to the user.

In some embodiments, the processor 102 may be configured to compare the association between the extracted at least one name feature and the selected image with at least one previously-made association. Responsive to determining that the association matches a previously-made association, the processor 102 may be configured to select, from the set of stored images, a different image associated with the name feature. For example, the processor 102 may be configured to select an image from the set of stored images that is different to an image selected previously. This can help to avoid confusion caused by using the same selected image to remember the identity of more than one person.

In a similar way, the processor 102 may attempt to avoid confusion caused by using the same or similar name features to remember the identity of multiple people. For example, the processor 102 may avoid the use of name features that have been used previously or name features that may be confused with name features that have been used previously. Thus, in some examples, the processor 102 may attempt to avoid any overlap between the extracted name feature and a name feature of a previously-made association. For example, if the processor 102 extracts the name feature "car" from the person's name, but it is recognized that the same name feature has been previously extracted and used in a different association for the same user, then the processor may extract a different name feature to be used. In this case, the processor 102 may be configured to generate a new association based on a different name feature of the person whose identity is to be remembered. This process may be repeated until a name feature has been extracted from the name that does not coincide (e.g. does not match or does not have a defined number of letters in common) with a name feature of a previously-made association.

The processor 102 may, in some examples, also attempt to avoid overlap between the extracted name feature and the subject matter of a previously-selected image. For example, the processor 102 may attempt to avoid the use of an extracted name feature "car" if it is determined that a previously-made association used an image of a car). In this case, the processor 102 may be configured to generate a new association based on a different name feature. This process may be repeated until a name feature has been extracted from the name that does not coincide with the subject matter of a previously-selected image of a previously-made association. Achieved by comparing the extracted name feature with tags or labels associated with each image in the set of stored images, for example.

In some examples, the processor 102 may attempt to avoid overlap between both (i) the extracted name feature and a name feature of a previously-made association and (ii) the extracted name feature and the subject matter of the selected image of a previously-made association. In this case, the processor 102 may be configured to generate a new association based on a different name feature. This process may be repeated until a name feature has been extracted from the name that does not coincide with either of a name feature, or the subject matter of a selected image of, a previously-made association.

The processor 102 may, in some examples, attempt to avoid overlap between the selected image and a selected image of a previously-made association (e.g. the selected image being an image of a car and a selected image of a previously-made association being an image of a car). In this case, the processor 102 may be configured to generate a new association based on a different selected image. This process may be repeated until an image has been selected from a set of stored images that does not coincide with a selected image of a previously-made association. The level of overlap taken into account when comparing images may vary based on user preferences or on other factors. For example, the processor 102 may be configured to avoid the use of an image of a car having the same color as a car in a previously-used image or to avoid the use of an image of a car of the same make or model as a car in a previously-used image. In some examples, known image similarity comparison techniques may be employed to ensure that images that are used in the associations are sufficiently different from previously-used images.

In some examples, the processor 102 may attempt to avoid overlap between the subject matter of a selected image and an extracted name feature of a previously-made association. For example, if the processor 102 selects an image of a car and it is determined that "car" was used as a previously-extracted name feature in a previously association, then the processor 102 may be configured to generate a new association based on a different selected image. This process may be repeated until an image has been selected from a set of stored images whose subject matter (e.g. a label or tag associated with the image) does not coincide with a name feature of a previously-made association.

The apparatus 200 may comprise features other than the processor 102. In some embodiments, the apparatus 200 may comprise a storage device for storing at least one of: the received image, the received name, the set of stored images, and the association of the at least one name feature with the selected image. The apparatus 200 may further comprise a user interface for presenting the selected first image and the name feature to the user. The user interface may be used for presenting other data to the user and, in some embodiments, may be used for receiving inputs from the user, such as a selection of an image or an association.

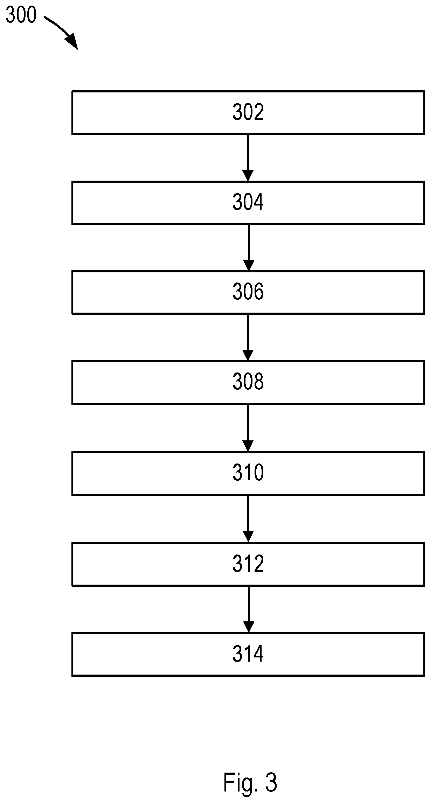

According to a second aspect, a method is provided. Fig. 3 is a flowchart of an example of a method 300, which may comprise a computer-implemented method. Steps of the method 300 may be performed using a processor, such as the processor 102 described herein. The computer-implemented method 300 comprises receiving 302 an image 104 of at least the face of a person whose identity is to be remembered; receiving 304 a name 106 of the person whose identity is to be remembered; extracting 306 at least one image feature 108 from the image of the face; selecting 308, from a set of stored images, a first image 110 associated with the at least one image feature; extracting 310 at least one name feature 112, 114, 116, 118 from the name; associating 312 the at least one name feature from the name with the selected first image; and providing 314 the selected first image and the name feature for presentation to a user.

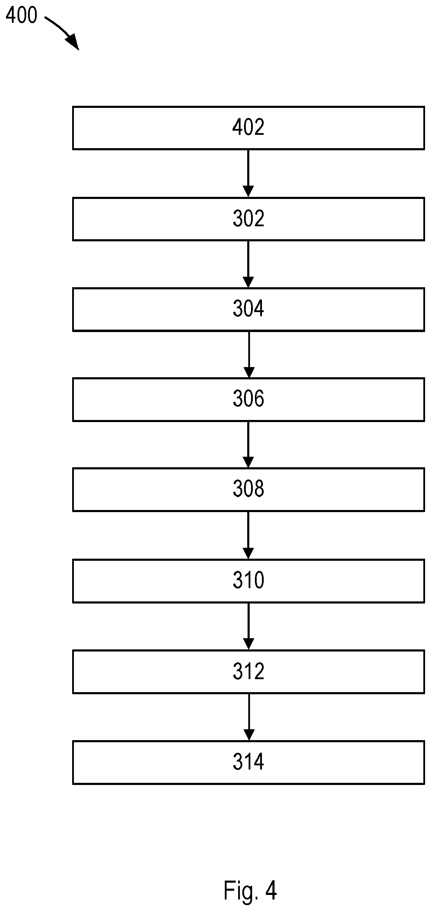

Fig. 4 is a flowchart of a further example of a method 400, which may comprise a computer-implemented method. In some embodiments, the computer-implemented method 400 may comprise one or more of the steps of the method 300 discussed above. In some embodiments, computer-implemented method 400 may comprise the step of receiving 402 a user input indicating a category of a plurality of categories, wherein each image in a set of stored images is associated with at least one of the plurality of categories. For example, the plurality of categories may be presented to the user via a user interface (e.g. via the device 120), and the user may select (e.g. via touching or clicking) a category of images that they feel will assist them most effectively at remembering the identity of the person. In some examples, selecting a first image associated with at least one image feature from an image of the face of a person whose identity is to be remembered may comprise selecting a first image from a category indicated in the user input.

Various techniques may be used for performing steps of the method 300, 400. In some embodiments, a role-based algorithm may be used for extracting image features and name features and/or for selecting an image from the set of stored images. In some embodiments, however, a predictive model may used to perform at least one of the steps of: extracting at least one image feature from an image of the face of a person whose identity is to be remembered, selecting, from a set of stored images, a first image associated with the at least one image feature, and extracting at least one name feature from the name of the person whose identity is to be remembered. For example, machine learning techniques may be used to train a model to identify and extract, from an image, an image feature that is likely to be considered particularly distinctive. Similarly, a model may be trained to identify and extract, from the person's name, a name feature that is likely to be considered particularly memorable. Similarly, a model may be trained to select, from the set of stored images, an image that is likely to be considered particularly memorable. Various machine learning techniques, classifiers and/or models/algorithms may be used including, for example, artificial neural networks, decision trees, support vector machines, regression analysis models, and the like.

In some embodiments, the step of extracting 306 at least one image feature from the image of the face of a person whose identity is to be remembered may comprise extracting a plurality of image features from the image of the face, presenting the plurality of image features to the user, receiving a user-selection of one of the plurality of image features, and using the user-selected image feature as the extracted image feature. In this way, the user is able to manually select a feature of the image that they consider to be most distinctive and/or memorable, to aid their memory of the identity of the person.

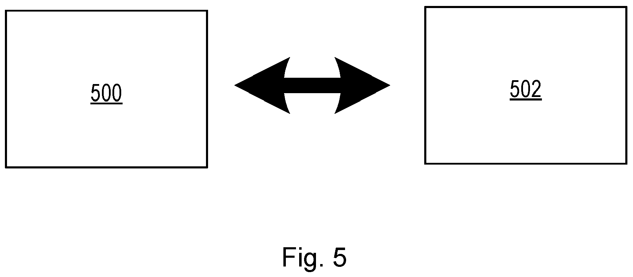

According to a third aspect, a computer program product is provided. Fig. 5 is a schematic illustration of an example of a computer-readable medium 500 in communication with a processor 502. According to various embodiments, a computer program product may comprise a non-transitory computer-readable medium 500, the computer-readable medium having computer-readable code embodied therein, the computer-readable code being configured such that, on execution by a suitable computer or processor 502, the computer or processor is caused to perform steps of the methods disclosed herein. The processor 502 may comprise, or be similar to, the processor 102 discussed above.

Embodiments disclosed herein provide a mechanism to aid the memory of a user trying to remember the identity of a person. For example, a user may find it difficult to remember a person's name, and to associate the name of the person with the face of the person. Embodiments of this disclosure extracts features from an image of the person and from the person's name, and uses those features to find alternative, more memorable images and more memorable words that can be associated with one another and can be more easily remembered by the user.

The processor 102, 502 can comprise one or more processors, processing units, multi-core processors or modules that are configured or programmed to control the apparatus 200 in the manner described herein. In particular implementations, the processor 102, 502 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (200) comprising:
a processor (102) configured to:
receive an image (104) of at least the face of a person whose identity is to be remembered;
receive a name (106) of the person whose identity is to be remembered;
extract at least one image feature (108) from the image of the face;
select, from a set of stored images, a first image (110) associated with the at least one image feature;
extract first name feature (112, 114, 116, 118) from the name; associate the first name feature with the selected first image; and
provide the selected first image and the first name feature for presentation to a user.

2. An apparatus (200) according to claim 1, wherein the processor (102) is further configured to:
select, from the set of stored images, a second image associated with the at least one image feature (108);
associate the first name feature (112, 114, 116, 118) with each of the first image (110) and the second image; and
provide the selected first image, the selected second image and the first name feature for presentation to the user.

3. An apparatus (200) according to claim 1, wherein the processor (102) is further configured to:
extract a second name feature from the name;
associate the first name feature (112, 114, 116, 118) and the second name feature with the selected first image (110); and
provide the selected first image, the first name feature and the second name feature for presentation to the user.

4. An apparatus (200) according to any of the preceding claims, wherein the images in the set of images are grouped according to a plurality of categories, and wherein the processor (102) is configured to select the first image (110) associated with the at least one image feature (108) by selecting the first image from a user-selected category of the plurality of categories.

5. An apparatus (200) according to any of the preceding claims, wherein the processor (102) is further configured to:
compare the association between the at least one name feature (112, 114, 116, 118) and the selected image (110) with at least one previously-made association; and
responsive to determining that the association matches a previously-made association, select, from the set of stored images, a different image associated with the name feature.

6. An apparatus (200) according to any of the preceding claims, wherein the processor (102) is configured to select the first image (110) associated with the at least one image feature (108) by searching the set of stored images to identify an image with a feature that matches, or is similar to, the at least one image feature.

7. An apparatus (200) according to any of the preceding claims, wherein each image in the set of stored images has at least one image feature (108) of a defined list of distinctive image features.

8. An apparatus (200) according to any of the preceding claims, wherein the at least one image feature (108) comprises a facial landmark feature.

9. An apparatus (200) according to any of the preceding claims, wherein the at least one name feature (112, 114, 116, 118) comprises one or more of: the whole name, part of the name, a homophone of the name or part of the name, a word that rhymes with the name or part of the name, and an anagram of the name or part of the name.

10. An apparatus (200) according to any of the preceding claims, further comprising:
a storage device for storing at least one of: the received image (104), the received name (106), the set of stored images, and the association of the at least one name feature (112, 114, 116, 118) with the selected image (110); and
a user interface for presenting the selected first image and the name feature to the user.

11. A computer-implemented method (300) comprising:
receiving (302) an image (104) of at least the face of a person whose identity is to be remembered;
receiving (304) a name (106) of the person whose identity is to be remembered;
extracting (306) at least one image feature (108) from the image of the face;
selecting (308), from a set of stored images, a first image associated with the at least one image feature;
extracting (310) at least one name feature (112, 114, 116, 118) from the name;
associating (312) the at least one name feature from the name with the selected first image; and
providing (314) the selected first image and the name feature for presentation to a user.

12. A computer-implemented method (300) according to claim 11, wherein a predictive model is used to perform at least one of the steps of: extracting (306) the at least one image feature, selecting (308) the first image, and extracting (310) at least one name feature.

13. A computer-implemented method (300) according to claim 11 or claim 12, further comprising:
receiving (402) a user input indicating a category of a plurality of categories, wherein each image in the set of stored images is associated with at least one of the plurality of categories;
wherein selecting a first image associated with the at least one image feature (108) comprises selecting a first image from the category indicated in the user input.

14. A computer-implemented method (300) according to any of claims 11 to 13, wherein extracting (306) at least one image feature (108) from the image comprises:
extracting a plurality of image features from the image of the face;
presenting the plurality of image features to the user;
receiving a user-selection of one of the plurality of image features; and
using the user-selected image feature as the extracted image feature.

15. A computer program product (500) comprising a non-transitory computer-readable medium, the computer-readable medium having computer-readable code embodied therein, the computer-readable code being configured such that, on execution by a suitable computer or processor (502), the computer or processor is caused to perform the method of any of claims 11 to 14.
